# FASCICULE DE BREVET EUROPEEN

(11) **EP 1 833 527 B1**
(45) Date de publication et mention de la délivrance du brevet: **15.06.2011**
(21) Numéro de dépôt: 05815187.9
(22) Date de dépôt: 07.11.2005
(51) Int. Cl.: A61M 1/00

(54) **DISPOSITIF D'ASPIRATION DE SÉCRÉTIONS NASALES**
VORRICHTUNG ZUM ABSAUGEN VON NASENSEKRETEN
NASAL SECRETION ASPIRATION DEVICE

(30) Priorité: 09.11.2004 FR 0411917
(43) Date de publication de la demande: 19.09.2007
(73) Titulaire: Ubimed L.L.C., Miami FL 33132 (US)
(72) Inventeur: BENSOUSSAN, José, F-69100 Villeurbanne (FR)
(74) Mandataire: Maureau, Philippe
(86) Numéro de dépôt international: PCT/FR2005/002770
(87) Numéro de publication internationale: WO 2006/051206

(56) Documents cités:
- EP-A- 1 051 984
- WO-A-02/13887
- DE-U1- 20 107 051
- US-A- 3 032 037

## Description

La présente invention concerne un dispositif d'aspiration de sécrétions nasales.

Ce dispositif peut être utilisé pour toute personne ayant des difficultés à se moucher.

Cependant, il est plus particulièrement destiné à moucher des enfants en bas âge ou des nourrissons ne sachant pas se moucher seul en dehors de l'éternuement ou de la déglutition par voies postérieures de leurs sécrétions nasales.

En cas de production exécive, les sécrétions nasales encombrent les fosses nasales, entraînant des grandes difficultés respiratoires pour les enfants en bas âge et les nourrissons étant donné que ceux-ci respirent spontanément par le nez.

Par ailleurs, la trompe d'Eustache séparant le cavum de l'oreille moyenne est très courte chez le nourrisson, un encombrement nasal favorise de ce fait la multiplication des otites moyennes aigües pouvant se transformer en otite chronique séro-muqueuse en cas d'encombrement chronique des fosses nasales avec hypertrophie secondaire des végétations adénoïdes.

De plus, les infections rhinopharyngées se communiquent à l'arbre trachéo-bronchique par inhalation.

La désobstruction des fosses nasales des nourrissons et des enfants en bas âge est donc essentielle sur le plan hygiénique et médical, complément indispensable des lavages de fosses nasales au sérum physiologique.

L'usage de solutions nasales antibiotiques est fortement déconseillée pour les nourrissons et les enfants en bas âge pour éviter d'augmenter les résistances bactériologiques.

Il est donc connu d'utiliser un dispositif d'aspiration de sécrétions nasales pour les enfants en bas âge et les nourrissons.

Plusieurs types de dispositifs sont connus, ils comportent généralement un embout nasal relié à une source de dépression.

Un premier type de dispositif connu est constitué d'une poire caoutchoutée reliée à un embout nasal.

Ce dispositif consiste à effectuer une pression sur la poire caoutchoutée afin de la vider de son air puis de placer l'embout nasal dans la fosse nasale d'un individu. Ensuite, en relâchant la pression sur la poire, celle-ci reprend sa forme originale et génère une dépression qui permet d'aspirer les sécrétions nasales.

Ce système génère une dépression brutale, très brève qui peut être désagréable pour un nourrisson ou un enfant en bas âge.

Pour des raisons d'hygiène, il est nécessaire de nettoyer et de stériliser la poire et l'embout nasal après chaque utilisation du dispositif, le dispositif n'étant pas à usage unique.

Or, la forme de la poire rend difficile un nettoyage et une stérilisation parfaite du dispositif.

Un second type de dispositif connu est constitué d'un embout buccal relié par un tuyau souple à un réservoir relié à un embout nasal.

Ce dispositif consiste à placer l'embout nasal dans la fosse nasale d'un individu puis d'aspirer les sécrétions nasales par l'embout buccal.

Ce type de dispositif est difficile à utiliser car il est long à mettre en oeuvre sur un nourrisson ou un enfant en bas âge qui a tendance à se débattre.

De plus, les sécrétions nasales remontent fréquemment dans l'embout buccal pouvant contaminer la personne qui aspire.

De même que précédemment, le dispositif doit être nettoyé et stérilisé après chaque usage.

Un troisième type de dispositif connu est constitué d'un embout nasal avec réservoir relié à une pompe électrique.

Ce dispositif consiste à placer l'embout nasal dans la fosse nasale d'un individu et de générer une dépression dans le réservoir à l'aide de la pompe électrique, ayant pour conséquence l'aspiration des sécrétions nasales.

De même que précédemment, le dispositif doit être nettoyé et stérilisé après chaque usage, celui-ci n'étant pas à usage unique.

De plus, dans ce type d'appareil, le réservoir n'est pas isolé de la pompe électrique, de ce fait les sécrétions nasales remontent fréquemment dans la pompe électrique qui est alors contaminée et peut se bloquer.

Le document DE 201 07 051 U1 décrit un dispositif d'aspiration de sécrétions nasales selon le préambule de la revendication 1.

La présente invention vise donc à remédier à ces inconvénients.

Le problème technique à la base de l'invention est la réalisation d'un dispositif d'aspiration de sécrétions nasales ne nécessitant pas un nettoyage et une stérilisation après chaque utilisation.

A cet effet, l'invention concerne un dispositif d'aspiration de sécrétions nasales selon la revendication 1.

Ainsi, sous l'effet d'une dépression créée dans la chambre de dépression, une dépression est créée dans l'embout nasal par déformation ou déplacement de l'élément non poreux déformable ou déplaçable. Cette dépression dans l'embout nasal assure l'aspiration des sécrétions.

De ce fait, lors d'une utilisation du dispositif, les sécrétions sont maintenues dans l'embout nasal formant réservoir qui est, pour des raisons d'hygiène, démonté et jeté après chaque usage.

Ainsi, le corps creux et la source de dépression ne sont pas contaminés par les sécrétions nasales et n'ont pas besoin d'être nettoyés et stérilisés après chaque usage du dispositif.

Dans un mode de réalisation de l'invention, l'élément obturant la seconde extrémité de l'embout nasal est constitué en une membrane imperméable déformable élastiquement telle qu'en latex ou en polychlorure de vinyle.

Avantageusement, l'élément obturant la seconde extrémité de l'embout nasal est constitué en matériau imperméable, telle qu'en papier enduit, plié à la façon d'un accordéon.

Dans un mode de réalisation de l'invention, la seconde extrémité de l'embout nasal est équipée d'un prolongement tubulaire cylindrique et axial à l'intérieur duquel est logé un piston déplaçable, sous l'effet d'une dépression, entre une position située au niveau de la seconde extrémité et une position éloignée de celle-ci. - - -

Avantageusement, l'embout nasal comporte deux pièces destinées à être fixées l'une à l'autre par emboîtement et en ce que la membrane imperméable est fixé sur l'embout nasal par coincement entre les deux pièces fixées par emboîtement.

Dans un mode de réalisation de l'invention, la membrane imperméable comporte un bourrelet périphérique destiné à être retenu dans un logement formé entre les deux pièces fixées par emboîtement.

Avantageusement, le bourrelet périphérique est destiné à coopérer avec un élément de retenue ménagé sur la paroi extérieure d'une des deux pièces formant l'embout nasal (2).

Dans un mode de réalisation de l'invention, les deux pièces formant l'élément nasal (2) comportent des moyens d'emboîtement comportant un cliquet et un logement de forme correspondante afin de former une liaison indémontable.

Avantageusement, l'embout nasal comprend à sa première extrémité, un clapet anti-retour évitant la vidange de l'embout nasal lorsqu'une dépression cesse d'être exercée sur l'élément déformable ou déplaçable obturant la seconde extrémité.

Dans un mode de réalisation de l'invention, le clapet anti-retour est constitué par une bille maintenue en appui sous l'action d'un ressort contre l'orifice de la première extrémité, lorsqu'aucune dépression n'est créée dans la chambre de dépression.

Avantageusement, les moyens de fixation de l'embout nasal sur le corps creux sont réalisés par vissage.

Dans un mode de réalisation de l'invention, les moyens de fixation de l'embout nasal sur le corps creux sont des moyens à encliquetage, à emboîtement ou de type baïonnette.

Avantageusement, le corps creux contient une pompe électrique, entraînée par un moteur électrique, destinée à créer une dépression dans la chambre de dépression.

Dans un mode de réalisation de l'invention, le moteur électrique est alimenté par des piles ou une batterie d'accumulateurs logée(s) dans le corps creux.

Avantageusement, la chambre de dépression est équipée d'un embout d'aspiration pour le montage d'un tube d'aspiration, par exemple, - buccale.

Dans un mode de réalisation de l'invention, la chambre de dépression est délimitée par une paroi susceptible de limiter la déformation de l'élément déformable obturant la seconde extrémité de l'embout nasal.

De toute façon l'invention sera bien comprise à l'aide de la description qui suit, en référence au dessin schématique indexé représentant, à titre d'exemples non limitatifs, plusieurs formes d'exécutions de ce dispositif d'aspiration de sécrétions nasales.
Figure 1 est une vue en coupe longitudinale d'une première forme d'exécution d'un dispositif d'aspiration de sécrétions nasales selon l'invention avant assemblage.
Figure 2 est une vue en coupe transversale selon la ligne II-II de figure 1.
Figure 3 est une vue en coupe longitudinale d'un dispositif selon la figure 1 en fonctionnement.
Figure 4 est une vue partielle en coupe longitudinale d'une deuxième forme d'exécution de l'invention.
Figure 5 est une vue partielle en coupe longitudinale d'une troisième forme d'exécution de l'invention.
Figure 6 est une vue partielle en coupe longitudinale d'une quatrième forme d'exécution de l'invention.
Figure 7 est une vue partielle en coupe longitudinale d'une cinquième forme d'exécution de l'invention.

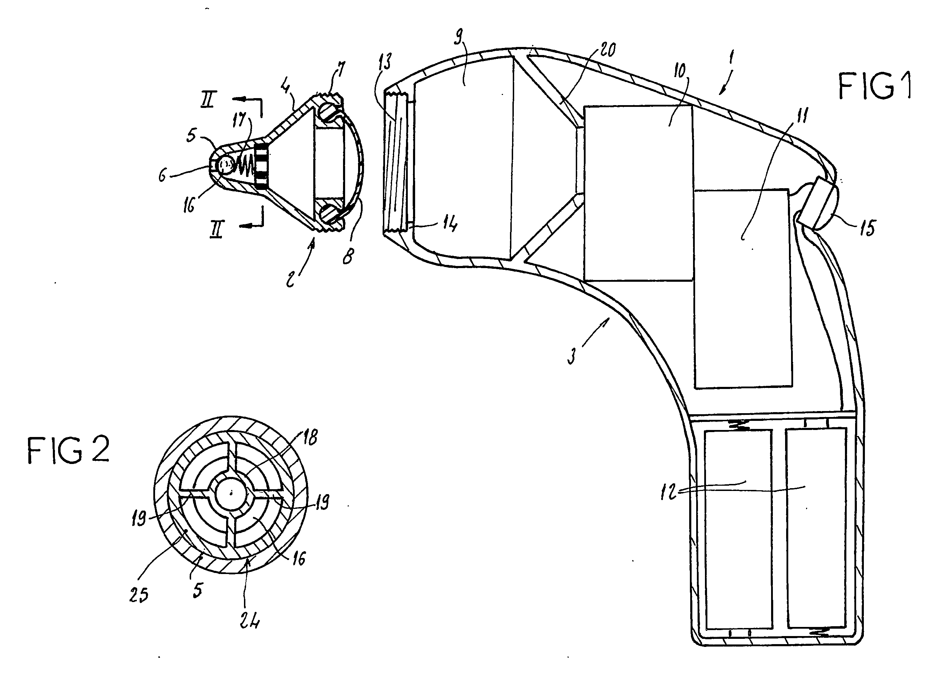

La figure 1 représente une première forme d'exécution d'un dispositif d'aspiration de sécrétions nasales 1 selon l'invention.

Le dispositif d'aspiration de sécrétions nasales 1 comprend un embout nasal 2 et un corps creux 3.

L'embout nasal 2 comporte une partie centrale creuse 4 de forme tronconique comportant, à son extrémité de plus faible section, une seconde partie tronconique creuse 5 de plus petit diamètre. La partie tronconique 5 comporte, à son extrémité de plus faible section, un orifice 6 et est adaptée pour être introduite dans le nez d'un individu.

La partie centrale tronconique 4 comporte, à son extrémité de plus grande section, une couronne 7 dont la surface extérieure est délimitée par un tronçon de surface cylindrique, la surface extérieure de la couronne étant filetée.

La couronne 7 est obturée de manière étanche par une membrane imperméable déformable élastiquement 8, de préférence en latex ou en polychlorure de vinyle.

La fixation de la membrane imperméable 8 sur l'embout nasal 2 est réalisée par complémentarité de formes.

Selon d'autres formes d'exécutions de l'invention, la fixation de la membrane imperméable 8 sur l'embout nasal 2 peut avantageusement être réalisée par collage ou par soudure ou bien les deux parties peuvent être moulées ou injectées ensemble.

Le corps creux 3 délimite une chambre de dépression 9 reliée à une pompe électrique 10 destinée à créer une dépression dans la chambre de dépression 9.

La pompe électrique 10 est entraînée par un moteur électrique 11 alimenté par des piles 12 logées dans le corps creux 3.

Le moteur électrique 11 est associé à des moyens de contrôle assurant un fonctionnement du moteur 11 avec temporisation, ainsi le moteur 11 ne peut fonctionner en continu que pendant une durée déterminée et ceci afin de limiter la dépression produite et d'éviter ainsi une détérioration du moteur 11.

Passée cette durée, le moteur 11 est arrêté par les moyens de contrôle, et ne peut être redémarré qu'après une seconde durée déterminée.

La chambre de dépression 4 comporte une ouverture 13 taraudée intérieurement de forme complémentaire de celle de la couronne 7 de l'embout nasal 2, destinée au vissage de l'embout nasal 2.

L'ouverture 13 du corps creux 3 est délimitée, dans le prolongement de son taraudage, par une butée périphérique 14 destinée à bloquer l'embout nasal 2 lors de son vissage dans l'ouverture 13.

Il en résulte que l'embout nasal 2 est monté de manière amovible sur le corps creux 3.

Un bouton poussoir 15 est prévu sur le corps creux 3 afin de permettre l'alimentation du moteur électrique 11 par les piles 12 dans sa position enfoncée.

Ainsi, lors d'une pression sur le bouton poussoir 15, la pompe - électrique 10 crée une dépression dans la chambre de dépression 9 qui entraîne la déformation de la membrane imperméable 8.

La déformation de la membrane imperméable 8 transmet la dépression dans l'embout nasal 2, il en résulte une aspiration des sécrétions nasales.

L'embout nasal 2 comprend dans la partie tronconique 5, un clapet anti-retour constitué par une bille 16 maintenue en appui sous l'action d'un ressort 17 contre l'orifice 6 de la partie tronconique 5 lorsqu'aucune dépression n'est créée dans la chambre de dépression 9.

Comme représenté à la figure 2, la zone de fixation du ressort 17 est constituée d'une pièce distincte 24 montée en force dans la partie tronconique 5 de l'embout nasal 2. La pièce distincte 24 est constituée d'une partie cylindrique et axiale 18 reliée par quatre pattes de matière 19 de même longueur à une partie cylindrique 25 de plus grand diamètre.

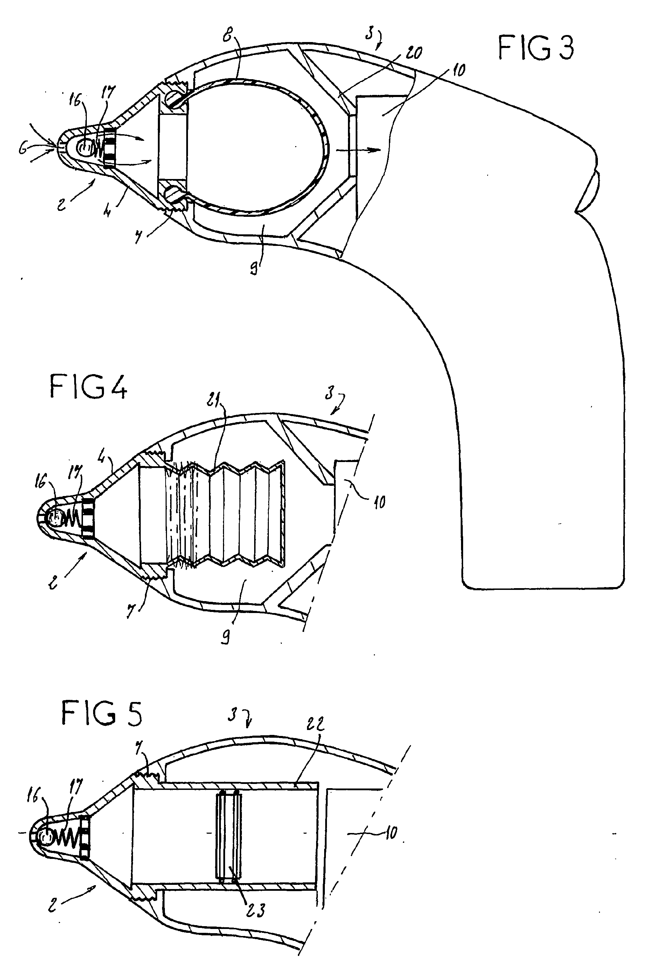

La figure 3 représente le dispositif selon la figure 1 en fonctionnement.

Lorsqu'une dépression est créée dans la chambre de dépression 9, la membrane imperméable 8 se déforme et transmet la dépression à l'embout nasal 2.

Il en résulte une compression du ressort 17 et de ce fait, la bille 16 n'obture plus l'orifice 6 de la partie tronconique 5 de l'embout nasal 2, permettant l'aspiration des sécrétions nasales.

Lorsque la dépression cesse d'être exercée sur la membrane imperméable 8, la bille 16, sous l'action du ressort 17, obture de nouveau l'orifice 6 de la partie tronconique 5, évitant ainsi la vidange de l'embout nasal 2.

Pour des raisons d'hygiène, l'embout nasal 2 est, de préférence, à usage unique.

Après utilisation du dispositif d'aspiration de sécrétions nasales 1, il est donc possible de dévisser l'embout nasal usagé 2 du corps creux 3, puis de le remplacer par un nouvel embout nasal 2.

Ainsi, le corps creux et la source de dépression ne sont pas contaminés par les sécrétions nasales et n'ont pas besoin d'être nettoyés et stérilisés après chaque usage du dispositif.

La chambre de dépression 9 est délimitée par une paroi 20, permettant la liaison entre la chambre de dépression 9 et la pompe électrique 10, susceptible de limiter la déformation de la membrane imperméable 8.

La figure 4 représente une deuxième forme d'exécution de l'élément obturant la couronne 7 de l'embout nasal 2.

Suivant cette forme d'exécution de l'invention, la couronne 7 est obturée de manière étanche par un élément déformable 21 constitué en matériau imperméable, telle qu'en papier enduit, plié à la façon d'un accordéon.

La fixation de l'élément déformable 21 sur l'embout nasal 2 est réalisée par collage.

La figure 5 représente une troisième forme d'exécution de l'élément obturant la couronne 7 de l'embout nasal 2.

Suivant cette forme d'exécution de l'invention, la couronne 7 est équipée d'un prolongement tubulaire cylindrique et axial 22 à l'intérieur duquel est logé un piston 23 déplaçable, sous l'effet d'une dépression, entre une position située au niveau de la couronne 7 et une position éloignée de celle-ci.

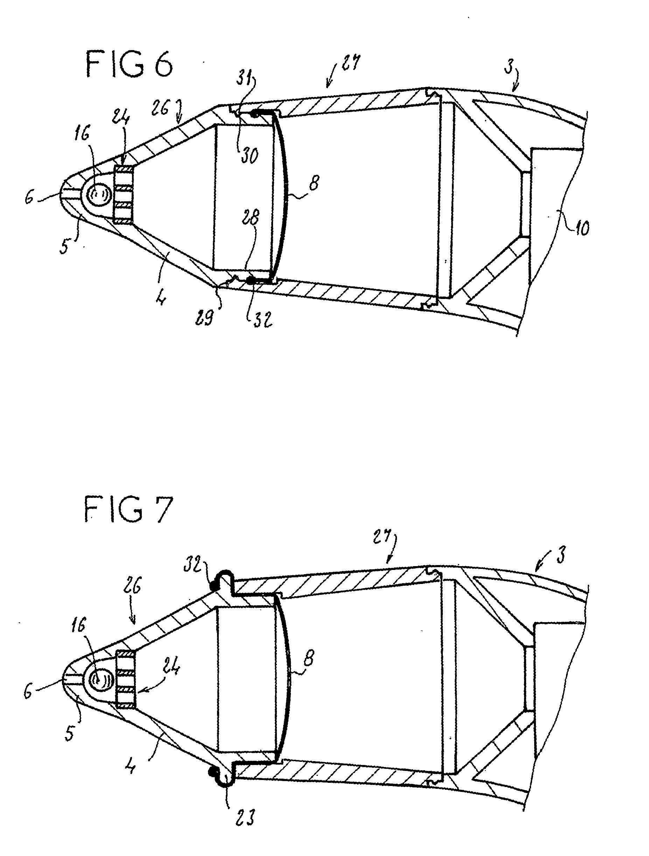

La figure 6 représente une quatrième forme d'exécution du dispositif d'aspiration de sécrétions nasales 1.

Dans cette forme d'exécution, les mêmes éléments portent les mêmes références.

A la différence de la première forme d'exécution, l'embout nasal 2 comporte deux pièces 26, 27 au lieu d'une.

La première pièce 26 comporte, de façon similaire à la première forme d'exécution, une partie centrale creuse 4 de forme tronconique, une seconde partie tronconique creuse 5, un orifice 6, une bille 16 et une pièce 24 montée en force dans la partie tronconique 5.

Dans cette forme d'exécution en particulier, la partie centrale creuse 4 de forme tronconique comporte, à son extrémité de plus grande section, un prolongement tubulaire 28 cylindrique et axial, équipé sur sa surface extérieure de moyens de fixation sur la seconde pièce 27.

Le diamètre extérieur du prolongement tubulaire 28 est plus faible que le diamètre extérieur de la partie centrale 4 à son extrémité de plus grande section. De ce fait, un rebord périphérique 29 est formé, servant de butée lors de l'emboîtement de la première pièce 26 dans la seconde pièce 27.

La seconde pièce 27 est creuse et de forme sensiblement tronconique. La seconde pièce 27 comporte une première extrémité ouverte équipée de moyens de fixation étanche et amovible dans l'ouverture 13 du corps creux 3 et une seconde extrémité de forme complémentaire de celle du prolongement tubulaire 28, destinée à l'emboîtement de la première pièce 26.

Un logement 30 est ménagé sur la surface extérieure du prolongement tubulaire 28 et un cliquet 31 de forme correspondante à celle du logement 30 est ménagé sur la surface intérieure de la pièce 27, le cliquet 31 étant destiné à s'emboîter dans le logement 30, ces deux éléments constituant des moyens d'emboîtement afin de former une liaison indémontable entre les pièces 26 et 27.

La membrane imperméable 8 comporte un bourrelet périphérique 32 destiné à être retenu dans un logement formé entre les deux pièces 26, 27 fixées par emboîtement, au niveau du prolongement tubulaire 28.

La membrane imperméable 8 est donc fixée sur l'embout nasal 2 par coincement entre les deux pièces 26, 27, et assure l'étanchéité entre les deux pièces 26, 27.

Suivant cette forme d'exécution de l'invention, l'embout nasal 2 comprend dans la partie tronconique 5, un clapet anti-retour constitué uniquement par la bille 16.

Lorsqu'une dépression cesse d'être exercée sur la membrane imperméable 8, la bille 16 obture l'orifice 6 de la partie tronconique 5, sous l'effet de la pression exercée par la membrane imperméable 8 qui a tendance à reprendre sa forme initiale, évitant ainsi la vidange de l'embout nasal 2.

Après utilisation du dispositif d'aspiration de sécrétions nasales 1, il est donc possible de déboîter l'embout nasal usagé 2 du corps creux 3, puis de le remplacer par un nouvel embout nasal 2.

Suivant cette forme d'exécution de l'invention, lors de l'utilisation du dispositif d'aspiration de sécrétions nasales, la membrane 8 se déforme et vient se plaquer contre la surface intérieure de la seconde pièce 27. De ce fait, lors du démontage de l'embout nasal 2, la membrane 8 n'est pas comprimée et les sécrétions nasales ne peuvent pas sortir de l'embout nasal 2, évitant ainsi une vidange partielle des sécrétions nasales.

La figure 7 représente une cinquième forme d'exécution du dispositif d'aspiration de sécrétions nasales 1.

Suivant cette forme d'exécution de l'invention, le bourrelet - périphérique 32 de la membrane imperméable 8 est destiné à coopérer avec un élément de retenue 33 ménagé sur la paroi extérieure de la première pièce 26, au niveau de l'extrémité de plus grande section de la partie centrale 4.

Les deux pièces 26, 27 ne sont pas fixées l'une à l'autre d'une façon indémontable mais simplement montées en force.

De ce fait, après utilisation du dispositif d'aspiration de sécrétions nasales 1, il est donc possible de déboîter l'embout nasal usagé 2 du corps creux 3, puis de désemmancher la première pièce 26 de la seconde pièce 27 afin de retirer la membrane imperméable 8 de la pièce 26 et de la jeter.

Suivant cette forme d'exécution de l'invention, seule la membrane imperméable 8 est à usage unique, l'embout nasal 2 devant être nettoyé et stérilisé après chaque usage du dispositif d'aspiration de sécrétions nasales.

Il va de soi que l'invention n'est pas limitée aux formes de réalisation, décrites ci-dessus à titre d'exemples, mais qu'elle en embrasse au contraire toutes les variantes de réalisation entrant dans le champ de protection défini par les revendications ci-annexées.

## Revendications

1. Dispositif d'aspiration de sécrétions nasales (1), comprenant :
- un corps creux (3) comportant une ouverture (13),
- et un embout nasal (2) comprenant une première extrémité ouverte (5) adaptée à l'orifice narinaire, destinée à être introduite dans le nez d'un individu et une seconde extrémité ouverte (7, 27) équipée de moyens de fixation étanche et amovible dans l'ouverture (13) du corps creux (3),
**caractérisé en ce que** le corps creux (3) délimite une chambre de dépression (9) pouvant être reliée à une source de dépression (10), la chambre de dépression (9) comportant l'ouverture (13), et **en ce que** la seconde extrémité (7, 27) est obturée par un élément non poreux déformable ou déplaçable (8, 21, 23) sous l'effet d'une dépression créée dans la chambre de dépression (9) par la source de dépression (10).

2. Dispositif d'aspiration de sécrétions nasales (1) selon la revendication 1, **caractérisé en ce que** l'élément obturant la seconde extrémité (7, 27) de l'embout nasal (2) est constitué en une membrane imperméable (8) déformable élastiquement, telle qu'en latex ou en polychlorure de vinyle.

3. Dispositif d'aspiration de sécrétions nasales (1) selon la revendication 1, **caractérisé en ce que** l'élément obturant la seconde extrémité (7) de l'embout nasal (2) est constitué en matériau imperméable (21), telle qu'en papier enduit, plié à la façon d'un accordéon.

4. Dispositif d'aspiration de sécrétions nasales (1) selon la revendication 1, **caractérisé en ce que** la seconde extrémité (7) de l'embout nasal (2) est équipée d'un prolongement tubulaire (22) cylindrique et axial à l'intérieur duquel est logé un piston (23) déplaçable, sous l'effet d'une dépression, entre une position située au niveau de la seconde extrémité (7) et une position éloignée de celle-ci.

5. Dispositif d'aspiration de sécrétions nasales (1) selon la revendication 2, **caractérisé en ce que** l'embout nasal (2) comporte deux pièces (26, 27) destinées à être fixées l'une à l'autre par emboîtement et **en ce que** la membrane imperméable (8) est fixé sur l'embout nasal (2) par coincement entre les deux pièces (26, 27) fixées par emboîtement.

6. Dispositif d'aspiration de sécrétions nasales (1) selon la revendication 5, **caractérisé en ce que** la membrane imperméable (8) comporte un bourrelet périphérique (32) destiné à être retenu dans un logement formé entre les deux pièces (26, 27) fixées par emboîtement.

7. Dispositif d'aspiration de sécrétions nasales (1) selon la revendication 6, **caractérisé en ce que** le bourrelet périphérique (32) est destiné à coopérer avec un élément de retenue (33) ménagé sur la paroi extérieure d'une des deux pièces (26, 27) formant l'embout nasal (2).

8. Dispositif d'aspiration de sécrétions nasales (1) selon l'une des revendications 5 à 7, **caractérisé en ce que** les deux pièces (26, 27) formant l'embout nasal (2) comportent des moyens d'emboîtement comportant un cliquet (30) et un logement (31) de forme correspondante afin de former une liaison indémontable.

9. Dispositif d'aspiration de sécrétions nasales (1) selon l'une quelconque des revendications 1 à 8, **caractérisé en ce que** l'embout nasal (2) comprend à sa première extrémité (5), un clapet anti-retour évitant la vidange de l'embout nasal (2) lorsqu'une dépression cesse d'être exercée sur l'élément déformable ou déplaçable (8, 21, 23) obturant la seconde extrémité (7, 27).

10. Dispositif d'aspiration de sécrétions nasales (1) selon la revendication 9, **caractérisé en ce que** le clapet anti-retour est constitué par une bille (16) maintenue en appui sous l'action d'un ressort (17) contre l'orifice (6) de la première extrémité (5), lorsqu'aucune dépression n'est créée dans la chambre de dépression (9).

11. Dispositif d'aspiration de sécrétions nasales (1) selon l'une quelconque des revendications 1 à 10, **caractérisé en ce que** les moyens de fixation de l'embout nasal (2) sur le corps creux (3) sont réalisés par vissage.

12. Dispositif d'aspiration de sécrétions nasales (1) selon l'une quelconque des revendications 1 à 10, **caractérisé en ce que** les moyens de fixation de l'embout nasal (2) sur le corps creux (3) sont des moyens à encliquetage, à emboîtement ou de type baïonnette.

13. Dispositif d'aspiration de sécrétions nasales (1) selon l'une quelconque des revendications 1 à 12, **caractérisé en ce que** le corps creux (3) contient une pompe électrique (10), entraînée par un moteur électrique (11), destinée à créer une dépression dans la chambre de dépression (9).

14. Dispositif d'aspiration de sécrétions nasales (1) selon la revendication 13, **caractérisé en ce que** le moteur électrique (11) est alimenté par des piles (12) ou une batterie d'accumulateurs logée(s) dans le corps creux (3).

15. Dispositif d'aspiration de sécrétions nasales (1) selon l'une quelconque des revendications 1 à 12, **caractérisé en ce que** la chambre de dépression (9) est équipée d'un embout d'aspiration pour le montage d'un tube d'aspiration, par exemple buccale.

16. Dispositif d'aspiration de sécrétions nasales (1) selon l'une quelconque des revendications 1 à 15, **caractérisé en ce que** la chambre de dépression (9) est délimitée par une paroi (20) susceptible de limiter la déformation de l'élément déformable (8,21) obturant la seconde extrémité (7, 27) de l'embout nasal (2).

## Claims

1. A device for suction of nasal secretions (1), comprising:
- a hollow body (3) including an aperture (13),
- and a nasal endpiece (2) comprising a first open end (5) adapted to the nose orifice, intended to be introduced into the nose of an individual and a second open end (7, 27) equipped with sealed and removable attachment means in the aperture (13) of the hollow body (3),
**characterized in that** the hollow body (3) delimits a depression chamber (9) which may be connected to a depression source (10), the depression chamber (9) including the aperture (13), and **in that** the second end (7, 27) is obturated by a deformable or displaceable non-porous element (8, 21, 23) under the effect of a depression generated in the depression chamber (9) by the depression source (10).

2. The device for suction of nasal secretions (1) according to claim 1, **characterized in that** the element obturating the second end (7, 27) of the nasal endpiece (2) consists in an elastically deformable impervious membrane (8) such as in latex or in polyvinyl chloride.

3. The device for suction of nasal secretions (1) according to claim 1, **characterized in that** the element obturating the second end (7) of the nasal endpiece (2) consists in an impervious material (21) such as a coated paper, folded in the fashion of an accordion.

4. The device for suction of nasal secretions (1) according to claim 1, **characterized in that** the second end (7) of the nasal endpiece (2) is equipped with a cylindrical and axial tubular extension (22) inside which is housed a piston (23) displaceable under the effect of a depression, between a position located at the second end (7) and a position away from the latter.

5. The device for suction of nasal secretions (1), according to claim 2, **characterized in that** the nasal endpiece (2) includes two parts (26, 27) intended to be attached to each other by their fitting to each other and **in that** the impervious membrane (8) is attached on the nasal endpiece (2) by blocking it between the two parts (26, 27) attached by fitting into each other.

6. The device for suction of nasal secretions (1) according to claim 5, **characterized in that** the impervious membrane (8) includes a peripheral bulge (32) intended to be retained in a housing formed between the two parts (26, 27) attached by fitting into each other.

7. The device for suction of nasal secretions (1) according to claim 6, **characterized in that** the peripheral bulge (32) is intended to cooperate with a retaining element (33) arranged on the outer wall of one of the two parts (26, 27) forming the nasal endpiece (2).

8. The device for suction of nasal secretions (1) according to one of claims 5 to 7, **characterized in that** the two parts (26, 27) forming the nasal endpiece (2) include fitting means including a pawl (30) and a housing (31) of a corresponding shape in order to form a connection which cannot be dismantled.

9. The device for suction of nasal secretions (1) according to any of claims 1 to 8, **characterized in that** the nasal endpiece (2) comprises at its first end (5), an anti-return valve avoiding emptying of the nasal endpiece (2) when a depression ceases being exerted on the deformable or displaceable element (8, 21, 23) obturating the second end (7, 27).

10. The device for suction of nasal secretions (1) according to claim 9, **characterized in that** the anti-return valve is formed by a ball (16) maintained under the action of a spring (17) so as to bear against the orifice (6) of the first end (5), when no depression is generated in the depression chamber (9).

11. The device for suction of nasal secretions (1) according to any of claims 1 to 10, **characterized in that** the means for attaching the nasal endpiece (2) onto the hollow body (3) are achieved by screwing.

12. The device for suction of nasal secretions (1) according to any of claims 1 to 10, **characterized in that** the means for attaching the nasal endpiece (2) onto the hollow body (3) are snap-on fastening, fitting or bayonet type fastening means.

13. The device for suction of nasal secretions (1) according to any of claims 1 to 12, **characterized in that** the hollow body (3) contains an electric pump (10) driven by an electric motor (11) intended to generate a depression in the depression chamber (9).

14. The device for suction of nasal secretions (1) according to claim 13, **characterized in that** the electric motor (11) is powered by battery cells (12) or a battery of accumulators housed in the hollow body (3).

15. The device for suction of nasal secretions (1) according to any of claims 1 to 12, **characterized in that** the depression chamber (9) is equipped with a suction endpiece for mounting a suction tube, for example a buccal tube.

16. The device for suction of nasal secretions (1) according to any of claims 1 to 15, **characterized in that** the depression chamber (9) is delimited by a wall (20) capable of limiting the deformation of the deformable element (8, 21) obturating the second end (7, 27) of the nasal endpiece (2).

## Patentansprüche

1. Vorrichtung zum Absaugen von Nasenschleim (1), umfassend:
- einen Hohlkörper (3) mit einer Öffnung (13),
- und eine Nasenkappe (2) mit einem ersten offenen Ende (5), das auf ein Nasenloch abgestimmt ist und seiner vorgesehenen Bestimmung nach in ein menschliches Nasenloch eingeführt wird, und einem zweiten, offenen Ende (7, 27), das mit Mitteln zum dichten und lösbaren Befestigen in der Öffnung (13) des Hohlkörpers (3) versehen ist,
**gekennzeichnet dadurch, dass** der Hohlkörper (3) eine Unterdruckkammer (9) umschließt, welche mit einer Unterdruckquelle (10) in Verbindung gebracht werden kann, wobei die Unterdruckkammer (9) die Öffnung (13) umfasst,
sowie **dadurch**, dass das zweite Ende (7, 27) durch ein nicht-poriges, unter Einwirkung des Unterdrucks, der in der Unterdruckkammer (9) durch die Unterdruckquelle (10) erzeugt wird, deformierbares oder bewegliches Element (8, 21, 23) verschlossen ist.

2. Vorrichtung zum Absaugen von Nasenschleim (1) nach Anspruch 1 **gekennzeichnet dadurch, dass** das das zweite Ende (7, 27) der Nasenkappe (2) verschließende Element aus einer undurchlässigen und elastisch verformbaren Membran (8), beispielsweise aus Latex oder Polyvinylchlorid, besteht.

3. Vorrichtung zum Absaugen von Nasenschleim (1) nach Anspruch 1 **gekennzeichnet dadurch, dass** das das zweite Ende (7) der Nasenkappe (2) verschließende Element aus einem undurchlässigen Material (21) wie beispielsweise beschichtetem, ziehharmonikaartig gefaltetem Papier besteht.

4. Vorrichtung zum Absaugen von Nasenschleim (1) nach Anspruch 1 **gekennzeichnet dadurch, dass** das zweite Ende (7) der Nasenkappe (2) mit einer röhrenartigen, zylindrischen und axial zu ihr verlaufenden Verlängerung (22), an deren Innenseite sich ein unter Einwirkung eines Unterdrucks zwischen einer Stellung in Höhe des zweiten Endes (7) und einer davon entfernten Stellung beweglicher Kolben (23) befindet.

5. Vorrichtung zum Absaugen von Nasenschleim (1) nach Anspruch 2 **gekennzeichnet dadurch, dass** die Nasenkappe (2) zwei Teile (26, 27) umfasst, deren Zweckbestimmung die Befestigung aneinander durch Ineinandergreifen derart ist, dass die undurchlässige Membran (8) auf der Nasenkappe (2) zwischen den fest ineinandergreifenden Teilen (26, 27) fest eingeklemmt ist.

6. Vorrichtung zum Absaugen von Nasenschleim (1) nach Anspruch 5 **gekennzeichnet dadurch, dass** die undurchlässige Membran (8) über einen umlaufenden Wulst (32) derart verfügt, dass dieser in einem Sitz zwischen den fest ineinandergreifenden Teilen (26, 27) festgehalten wird.

7. Vorrichtung zum Absaugen von Nasenschleim (1) nach Anspruch 6 **gekennzeichnet dadurch, dass** der umlaufende Wulst (32) dazu bestimmt ist, mit einem Rückhalteelement (33), das sich an der äußeren Wandung eines der beiden Teile (26,27) befindet, welche die Nasenkappe (2) bilden, zusammenzuwirken.

8. Vorrichtung zum Absaugen von Nasenschleim (1) nach einem der Ansprüche 5 bis 7 **gekennzeichnet dadurch, dass** die beiden Teile (26, 27), welche die Nasenkappe (2) bilden, eine Rastklinke (30) und einen entsprechenden Sitz (31) derart tragen, dass eine nicht lösbare, formschlüssige Steckverbindung ausgebildet wird.

9. Vorrichtung zum Absaugen von Nasenschleim (1) nach irgendeinem der Ansprüche 1 bis 8 **gekennzeichnet dadurch, dass** die Nasenkappe (2) an ihrem ersten Ende (5) ein Rückschlagventil trägt, welches das Entleeren der Nasenkappe (2) verhindert, wenn der Unterdruck auf das deformierbare oder bewegliche Element (8, 21, 23), welches das zweite Ende (7, 27) verschließt, verschwindet.

10. Vorrichtung zum Absaugen von Nasenschleim (1) nach Anspruch 9 **gekennzeichnet dadurch, dass** die Rückschlagklappe aus einer Kugel (16) besteht, die unter der Einwirkung einer Feder (17) gegen das Mundstück (6) des ersten Endes (5) gedrückt wird, sobald in der Unterdruckkammer (9) kein Unterdruck mehr erzeugt wird.

11. Vorrichtung zum Absaugen von Nasenschleim (1) nach irgendeinem der Ansprüche 1 bis 10 **gekennzeichnet dadurch, dass** die Mittel zur Befestigung der Nasenkappe (2) auf dem Hohlkörper (3) eine Form von Gewinde aufweisen.

12. Vorrichtung zum Absaugen von Nasenschleim (1) nach irgendeinem der Ansprüche 1 bis 10 **gekennzeichnet dadurch, dass** die Mittel zur Befestigung der Nasenkappe (2) auf dem Hohlkörper (3) formschlüssige Steckverbindungen oder Bajonettverschlüsse mit Rastwirkung sind.

13. Vorrichtung zum Absaugen von Nasenschleim (1) nach irgendeinem der Ansprüche 1 bis 12 **gekennzeichnet dadurch, dass** der Hohlkörper (3) eine elektrische Pumpe (10) beinhaltet, welche durch einen Elektromotor (11) angetrieben wird und dazu bestimmt ist, in deren Unterdruckkammer (9) einen Unterdruck zu erzeugen.

14. Vorrichtung zum Absaugen von Nasenschleim (1) nach Anspruch 13 **gekennzeichnet dadurch, dass** der Elektromotor (11) seine Betriebsspannung aus Batterien (12) oder einer Anzahl von Akkumulatoren bezieht, welche sich im Hohlkörper (3) befinden.

15. Vorrichtung zum Absaugen von Nasenschleim (1) nach irgendeinem der Ansprüche 1 bis 12 **gekennzeichnet dadurch, dass** die Unterdruckkammer (9) mit einem Ansaugende für das Anbringen eines Ansaugrohrs, beispielsweise buccal, vorgesehen ist.

16. Vorrichtung zum Absaugen von Nasenschleim (1) nach irgendeinem der Ansprüche 1 bis 15 **gekennzeichnet dadurch, dass** die Unterdruckkammer (9) durch eine Wandung (20) begrenzt ist, welche geeignet ist, die Verformung des deformierbaren Elements (8,21), welches das zweite Ende (7,27) der Nasenkappe (2) verschließt, zu begrenzen.
